# EUROPÄISCHE PATENTSCHRIFT

(11) **EP 1 348 117 B1**
(45) Veröffentlichungstag und Bekanntmachung des Hinweises auf die Patenterteilung: **09.11.2005**
(21) Anmeldenummer: 02803344.7
(22) Anmeldetag: 14.08.2002
(51) Int. Cl.: G01N 15/14, G01N 33/543

(54) **VERFAHREN ZUM FESTSTELLEN VON SUBSTANZEN IN EINER FLÜSSIGEN ODER GASFÖRMIGEN PROBE**
METHOD FOR THE DETERMINATION OF SUBSTANCES IN A LIQUID OR GASEOUS SAMPLE
PROCEDE DE DETECTION DE SUBSTANCES DANS UN ECHANTILLON LIQUIDE OU GAZEUX

(30) Priorität: 20.11.2001 DE 10156612
(43) Veröffentlichungstag der Anmeldung: 01.10.2003
(73) Patentinhaber: Klotz, Markus, 75378 Bad Liebenzell (DE)
(72) Erfinder: Klotz, Markus, 75378 Bad Liebenzell (DE)
(74) Vertreter: Steimle, Josef
(86) Internationale Anmeldenummer: PCT/EP2002/009123
(87) Internationale Veröffentlichungsnummer: WO 2003/044493

(56) Entgegenhaltungen:
- US-A- 4 747 685
- US-A- 4 879 211
- US-A- 4 999 513
- US-A- 6 159 748

## Beschreibung

Die Erfindung betrifft ein Verfahren zum Feststellen von Substanzen in einer flüssigen oder gasförmigen Probe nach dem Oberbegriff des Patentanspruchs 1.

Bei diesen Substanzen bzw. Substanz-Spezifika handelt es sich zum einen um ggf. nur molekulare Stoff- bzw. Materie-Mengen, zum anderen - im Bereich der Biologie und Medizin - um ebenfalls molekulare krankheitserregende Keime, also Bakterien, Viren, Pilze, und Parasiten, aber auch z. B. um Antikörper, Proteine, genetische Informationen usw. im Mikro- bis Nanometerbereich.

In diesem Bereich verstärkt sich z. B. aufgrund zunehmender Kenntnis der genetisch kodierten Information und der Fortschritte in der Erforschung biologisch-medizinischer Systeme der Bedarf an leistungsfähigen Diagnose- bzw. Analyseverfahren zum Feststellen diesbezüglicher Substanzen in entsprechenden Proben.

Die bislang z. B. dafür eingesetzten miniaturisierten Mikrotiterplatten mit z. B. bis zu 1536 Probenkammern wie auch die hochintegrierten, so genannten "BioChips" mit bis zu 100.000 Matrixelementen, erfordern jeweils zeit- und geräteaufwändige Vorbereitungsmaßnahmen. So müssen im ersten Fall zwei oder mehrere verschiedene chemische Reagenzien in eine Probekammer dispensiert und die sich ggf. anschließende, zu einer Immobilisierung eines Reaktionspartners auf der Kammeroberfläche führende Interaktion zwischen den Reaktionspartnern, die so genannte Hybridisierung, durch standardisierte analytische Messtechniken (Absorption, Fluoreszenz, Luminizenz usw.) erfasst werden.

Beim "BioChip" werden diskrete, durch aufwändige, z. B. drucktechnische Verfahren, z. B. mit Nukleinsäuren oder Proteinen beladene Matrixelemente mit der zu untersuchenden Probe inkubiert und die Hybridisierungs-Reaktion durch Erwärmen ausgelöst. Allein für diese Vorbereitung ist entsprechende Erfahrung und eine kostspielige Geräteausrüstung notwendig. Die anschließenden Reaktionen sind nicht direkt beeinflussbar und somit ist eine Qualitätskontrolle nicht möglich.

Aufgabe der Erfindung ist es, ein Verfahren bereitzustellen, mit dem ohne spezielle Erfahrungen, insbesondere aber ohne großen Vorbereitungsaufwand und aufwändige Gerätetechnik, ggf. auch mobil an beliebigen Orten, die in einer flüssigen oder gasförmigen Probe vermutlich vorhandenen Substanzen der zuvor erwähnten Art in einem einzigen Analysevorgang festgestellt werden können.

Diese Aufgabe wird durch ein Verfahren nach Patentanspruch 1 gelöst. Vorteilhafte Weiterbildungen sind Gegenstand der Unteransprüche.

Die Erfindung geht einerseits aus von im Handel erhältlichen, vorzugsweise kugelförmigen, mit einer Größenverteilung von wenigstens < 1% annähernd homogen hergestellten so genannten Mikropartikeln, vorzugsweise aus Latex (= vernetztes Polystyrol, ggf. auch Polypropylen), mit einem in Größenklassen gestaffelten Durchmesser von z. B. 2 µm bis 150 µm, der aber auch im Millimeter- wie auch im Nanometer-Bereich liegen kann. Diese "nach Katalog" bestellbaren Mikropartikel können zudem - ebenfalls gemäß Bestellung - bereits mit so genannten Bindungspartnern präpariert sein, die in der Lage sind, jeweils eines der zuvor erwähnten, die festzustellende Substanz repräsentierendes Spezifikum unlösbar an sich zu binden. Auf diese Weise entsteht eine Art Schicht auf dem einzelnen Mikropartikel, wodurch dessen Größe zunimmt, wenn auch überwiegend nur im Molekularbereich.

Andererseits sind hochpräzise, vorzugsweise mit einem laser-optischen Verfahren arbeitenden Messgeräte im Handel, mit denen z. B. die Größe solcher Mikropartikel, aber auch deren besagte molekulare Größenzunahme aufgrund solcher Bindungsreaktionen zumindest in flüssigen Substraten mit einer sehr hohen Genauigkeit der Größenbestimmung (deutlich besser als 2%) gemessen werden kann. Bei einer Mikropartikeldichte bis 200.000 Mikropartikel/ml können demgemäß 20 bis 100 und mehr unterschiedlich große Mikropartikel bzw. unterschiedliche Mikropartikel-Größenklassen unterschieden werden.

Vor diesem Hintergrund basiert die Erfindung auf der Idee, das Vorhandensein von in einer Probe vermuteten Substanzen dadurch nachzuweisen, dass jeder dieser Substanzen zunächst eine bestimmte definierte Größenklasse derartiger Mikropartikel sozusagen abstrakt zugeordnet wird. Wird dann eine Mischung aus diesen so ausgewählten, größenklassenweise substanzspezifisch mit den jeweiligen Substanzen entsprechenden Bindungspartnern präparierten, d. h. besetzten Mikropartikeln in die zu untersuchende Probe gegeben, so binden die einzelnen Bindungspartner der Mikropartikel einer ganz bestimmten Größenklasse jeweils zuordnungsgemäß ein Spezifikum der tatsächlich in der Probe vorhandenen Substanz an sich, wodurch die Größe der so hybridisierten Mikropartikel zunimmt.

Werden daraufhin in einer Probe größere Mikropartikel gemessen als es den jeweils gemäß abstrakt vorgenommener Zuordnung vorgegebenen Ausgangs-Größenklassen entspricht, so steht fest, dass die diesen zugeordneten Substanzen auch tatsächlich in der Probe enthalten sind. Es wird also die Probe nicht direkt auf das Vorhandensein von bestimmten Substanz-Spezifika analysiert, sondern es wird indirekt über die Feststellung einer Größenzunahme bei Mikropartikeln einer definierten Ausgangsgröße auf sie geschlossen. Bei richtiger Auswahl der Mikropartikel-Größenklassen, d. h. ihrer jeweiligen Abstände voneinander, kommt es also auf die absolute Größenzunahme der einzelnen Mikropartikel nicht an.

Im Hinblick auf die Eindeutigkeit dieser Messergebnisse sollten sich dementsprechend die jeweils auszuwählenden Mikropartikel-Größenklassen in Bezug auf die Größenordnung der festzustellenden Substanzen bzw. deren Spezifika deutlich voneinander unterscheiden. So kann vermieden werden, dass etwa durch das Binden von einer übermäßig großen Anzahl von Spezifika an einen Mikropartikel ggf. die nächst größere, einer anderen in der Probe vermuteten Substanz zugeordnete Mikropartikel-Größenklasse erreicht wird.

Andererseits ist bei der abstrakten Gößenklassenzuordnung auch zu beachten, dass die Mikropartikel-Größenklassen so gewählt werden, dass durch das Binden von Substanz-Spezifika an die Mikropartikel der jeweils zugeordneten Mikropartikel-Größenklasse die Mikropartikel-Größe zumindest laseroptisch messbar zunimmt.

Zum Messen der Größenzunahme von Mikropartikeln wird die Probe nach der Hybridisierungsphase in einer Messküvette durch einen Messkanal geleitet, wobei ein den Messkanal beleuchtender Lichtstrahl in Abhängigkeit von der Größe der durch den Messkanal nacheinander hindurchtretenden hybridisierten bzw. nicht hybridisierten Mikropartikel unterschiedlich stark geschwächt wird, was von einem Lichtschwächungs-Messsensor detektiert und in ein der jeweiligen Schwächung entsprechendes Mikropartikel-Größensignal, z. B. in Form eines Strom- oder Spannungssignals, umgesetzt wird. Den Messkanal passieren die Mikropartikel in der zu analysierenden Probe in einer für eine notwendige Messsicherheit ausreichenden Vereinzelung, was bei der erwähnten Mikropartikeldichte von 200.000 Mikropartikel/ml gewährleistet ist.

### Hierzu ein Beispiel:

Werden z. B. Mikropartikel der Größe 2 µm mit Erkennungssequenzen (= Antikörpern) für eine Rötelinfektion, andere Mikropartikel der Größe 50 µm mit Molekülen für Hepatits-A und weitere Mikropartikel der Größe 100 µm mit Sequenzen zur Erkennung des HIV-Virus wie erläutert präpariert, dann kann mit einer einzigen Probenanalyse, bei der erfindungsgemäß hybridisierte Mikropartikel zur Ermittlung einer Größenzunahme gemessen werden, festgestellt werden, ob ein Mensch Röteln hatte und ob zudem eine der beiden anderen Infektionen vorliegt.

Das erfindungsgemäße Verfahren zeichnet sich durch folgende Eigenschaften aus:
- kein kompliziertes Beladen ("Spotten") von Matrixstrukturen mit diskreten Matrixelementen, wie bei den schon erwähnten "BioChips", da erfindungsgemäß unterschiedliche Mikropartikelgrößen die Matrixstruktur ersetzen, so dass eine kompliziert auszuführende strukturelle/räumliche Zuordnung entfällt;
- es sind unterschiedliche Mikropartikel-Materialien einsetzbar, wie z. B. Latex, Kunststoff, Glas oder auch Metall; folglich können unterschiedliche Mikropartikelmaterialien für spezifische Beladungs- und/oder Bindungsbedingungen (Hybridisierungsbedingungen) der auf die Mikropartikel aufzubringenden Reaktionspartner, die jeweils die Bindung von z. B. Molekülen einer der in der zu analysierenden Probe zu identifizierenden Substanz an die unterschiedlich großen Mikropartikel sicherstellen, ausgewählt werden;
- die Mikropartikel der einzelnen Größenklassen lassen sich, im Gegensatz z. B. zu den "BioChip"-Präparierungen, separat in so genannten "Ansätzen" in zudem automatisch ablaufenden Vorgängen präparieren, was eine bessere Qualität gewährleistet, die folglich auch kontrollierbar ist; dieses substanzbezogene Vorbereiten von Mikropartikeln wird inzwischen als Dienstleistung von entsprechenden Unternehmen angeboten, so dass für jede durchzuführende Probenanalyse die dafür erforderlichen präparierten Mikropartikel bereits "nach Katalog" bestellt werden können, was die Analysen-Vorbereitung ganz entscheidend vereinfacht, verbilligt und beschleunigt, da nach wenigen Handgriffen zum Einbringen der präparierten Mikropartikel mehrerer Größenklassen, üblicherweise manuell mit einer Pipette aus z. B. einem "Vorratsglas", die Probenanalyse durchgeführt werden kann;
- auf diese Weise ist auch die Qualitätskontrolle der einzelnen Chargen gewährleistet und kann die Zusammensetzung der Analyse-"Ansätze" individuell angepasst werden;
- die "Ansätze" können in beliebigen Mikrobehältern erfolgen, die gleichzeitig auch die Messküvette bilden können;
- die für die Analyse einer Probe mit vermuteten Substanzen spezifischen Analysen-"Ansätze" können fallbezogen beliebig zusammenstellbare Mischungen von Mikropartikeln unterschiedlicher Mikropartikel-Größenklassen enthalten, die auch, wie schon erwähnt, z. B. einfach mit der Pipette z. B. aus dem Mikropartikel-"Vorratsglas" in die Probe gegeben werden können;
- die Hybridisierung der ausgewählten Mikropartikel mit Molekülen der in der Probe enthaltenen Substanzen erfolgt in einer normalen Küvette, z. B. in der Messküvette, entsprechend den Temperierbedingungen;
- in der "BioChip"-Technologie erforderliche Waschvorgänge fallen nicht an; dennoch erfolgende sind viel einfacher und auch automatisiert durchführbar;
- der in der (Mess-) Küvette enthaltene "Ansatz" kann aufwandsvereinfachend direkt analysiert werden;
- in den zu analysierenden "Ansätzen" können ohne "Chips" und geometrische Zuordnung immobilisierte Reaktionspartner z. B. medizinisch-diagnostisch relevante Reaktionen (auf DNA- oder Proteinbasis) schnell, flexibel, kostengünstig und ohne hohen gerätetechnischen Aufwand (keine Pipettiervorrichtung, keine Roboter für das Auftragen der Reaktionspartner, keine "BioChip"-Reader) mit Möglichkeiten der Qualitätskontrolle (auch für Zertifizierungs-Anforderungen) nachgewiesen werden;
- es können sämtliche unter Messbedingungen in der Messküvette transparenten Flüssigkeiten oder auch Gase auf in ihnen enthaltene - molekulare - Substanzen analysiert werden, so z. B. auch Öl auf ggf. darin enthaltenes Wasser oder auf Zusätze, wie z. B. Additive usw., aber auch Chemikalien;
- dementsprechend können auch gasförmige Medien als Probensubstrat für erfindungsgemäße Substanzanalysen benutzt werden;
- Anwender können sich mit entsprechenden Chargen von in präzisen Größenklassen < 1% vom Hersteller erhältlichen Mikropartikeln selbst Analysekits zusammenstellen bzw., wie schon erwähnt, auch fertige, unmittelbar einsetzbare Mikropartikelmischungen kaufen; dadurch vereinfachen sich Diagnoseverfahren in der molekularen Medizin, was zur Verbreitung derartiger diagnostischer Tests bzw. Analysen und der damit verbundenen diagnostischen Aussagen zur Sicherheit von Arzt und Patient führt;
- alle prozessbedingten Schritte können in Mikroküvetten auf dem üblichen nasschemischen Weg durchgeführt werden;
- die gerätetechnische Realisierung der erfindungsgemäßen Probenanalyse-Verfahrenstechnik ermöglicht kompakte Geräte, die leicht transportabel sind und so vorteilhaft auch mobil für Substanzfeststellungen vor Ort einsetzbar sind, z. B. vom Arzt bei Patienten-Hausbesuchen oder von Krankenschwestern bei Kontrolluntersuchungen am Krankenbett; dabei können die Analyseergebnisse, also welche Substanzen in der untersuchten Probe festgestellt worden sind, durch Angabe von deren Namen, wie z. B. bei Krankheiten, z. B. auf einem entsprechenden Display, angezeigt werden, wofür z. B. eine Software die vorgenommene, eingangs in das Analysegerät einzugebende Zuordnungs-Auswahl zwischen Mikropartikel-Größenklasse und vermuteter Substanz aufgrund der ggf. jeweils gemessenen Mikropartikel-Größenzunahmen entsprechend auswertet.

Sofern es notwendig erscheint, können vor dem Durchleiten der Probe durch den Messkanal in einer Art Waschvorgang nicht an Bindungspartner gebundene Substanz-Spezifika aus der Probe entfernt werden, um ggf. so eine größere Reinheit der Probe für den Messvorgang zu erreichen.

Für den Fall, dass durch das Binden von Substanz-Spezifika in der Hybridisierungsphase an die Bindungspartner von Mikropartikeln einer bestimmten Größenklasse keine für eine zuverlässige Größenmessung ausreichend große Größenzunahme dieser Mikropartikel erreicht werden kann, könnte eine zusätzliche Größenzunahme dieses bereits von einem Mikropartikel und daran gebundener Substanz-Spezifika gebildeten Konstrukts wie folgt erreicht werden: Für eine weitere Hybridisierungsphase werden entsprechende substanzspezifische Bindungspartner für die diesbezügliche Mikropartikel-Größenklasse in die Probe gegeben, die jetzt aber von den bereits von den Bindungspartnern der zugehörigen Mikropartikel gebundenen Spezifika gebunden werden und so eine Art zweite Schicht auf diesen Mikropartikeln bilden.

Zum Feststellen von in einer Probe vermuteten Krankheitserregern werden zu deren Binden an die dafür ausgewählten Mikropartikel als Bindungspartner mit Vorteil diesen entsprechende Antikörper benutzt.

Umgekehrt werden zur Feststellung von Antikörpern in einer Probe als Mikropartikel-Bindungspartner mit Vorteil diesen entsprechende Krankheitserreger benutzt.

Für den Fall, dass die Ergebnisse der Größenzunahme-Messung von hybridisierten Mikropartikeln einer bestimmten Größenklasse als nicht ausreichend signifikant gegenüber denjenigen anderer Größenklassen sein sollten, um daraus auf das Vorhandensein einer bestimmten Substanz in der untersuchten Probe schließen zu können, kann die Größenzunahme-Messung mit Vorteil durch eine weitere hybridisierungsabhängig erfassbare Messgröße ergänzt werden. Diese kann z. B. aus einem von hybridisierten Mikropartikeln aufgrund entsprechender Präparierung ausgesendeten Licht gewonnen werden.

Ein weiterer Grund für die Kombination zwei derartiger Messungen für Probenanalysen kann sich z. B. auch aufgrund der Abstände der verfügbaren Mikropartikel-Größenklassen voneinander als notwendig erweisen, oder aber, anders herum, um bei diesen geringere Abstände voneinander zu ermöglichen,

Hierzu können die bereits größenklassenabhängig mit substanzspezifischen Bindungspartnern präparierten Mikropartikel zusätzlich noch mit einem zweiten anderen, evtl. Fluoreszenz-gelabelten Reaktionspartner, einem so genannten "Marker", auf ihrer Oberfläche beladen werden. Ggf. überschüssige Marker werden, wie ggf. schon bei nicht gebundenen Substanz-Spezifika, in einem im Gegensatz z. B. zu "BioChips" einfach durchzuführenden, ohne weiteres ebenfalls automatisierbaren Art Waschvorgang entfernt.

Bei der zweiten Messgröße kann es sich z. B. um ein mittels eines Anregungs-Lichtstrahls, vorzugsweise ebenfalls eines Laserlichtstrahls, erzeugtes Fluoreszenzlicht handeln mit gegenüber dem Anregungslicht anderer Wellenlänge, das von einem entsprechenden zweiten Messsensor erfasst wird.

Mit diese beiden, in einer aufeinander bezogenen Auswertung miteinander verknüpften Signale würde dann festgestellt werden können, dass eine bestimmte, entsprechend der Mikropartikel-Präparierung in der Probe vermutete Substanz auch tatsächlich darin enthalten ist. Hier würde somit erst das zum Mikropartikel-Größensignal hinzukommende Fluoreszenzlicht-Signal die Schlussfolgerung erlauben, dass ein Mikropartikel bestimmter Größenklasse tatsächlich mit Spezifika der ihm zunächst abstrakt über seine Größenklasse, dann konkret über die größenklassenspezifische Präparierung mit den diesbezüglichen Bindungspartnern zugeordneten Substanz hybridisiert worden ist, wobei über das Mikropartikel-Größensignal sofort feststeht, um genau welche Substanz es sich handelt.

Zu diesem Zweck müssten die beiden Messsensoren in der Lage sein, die Größe eines einzelnen Mikropartikels mit größtmöglicher Genauigkeit und das von ihm bei Beleuchtung ausgehende, ggf. bis zu drei und mehr unterschiedliche Frequenzen aufweisende Fluoreszenzlicht zu erfassen, dies ggf. auch simultan für die anschließende Auswertung.

Diese beiden Messgrößen können, statt ggf. simultan, auch in kürzesten zeitlichen Abständen nacheinander detektiert werden. Hierzu könnten z. B. zwei verschiedene Laserlichtstrahlen in geringen räumlichen Abstand voneinander, z. B. untereinander, auf den Messkanal der Messküvette gerichtet werden, so dass die Mikropartikel zunächst den einen und in einem der räumlichen Distanz der Laserstrahlen untereinander entsprechenden, ermittelbaren Zeitabstand den zweiten Laserstrahl, ggf. anderer Wellenlänge als der erste, passieren.

Die so von einem hybridisierten Mikropartikel nacheinander ermittelten beiden Messgrößen "Mikropartikelgröße" und "Fluoreszenzlicht-Aussendung" könnten dann trotz der zeitlich verschobenen Erfassung für die Auswertung zur Substanzfeststellung einander zugeordnet werden.
Im Hinblick auf ein deutlich erfassbares Fluoreszenzlicht-Signal wäre es vorteilhaft, wenn die der Probe jeweils beigefügten Mikropartikel mit jeweils möglichst vielen Markern beladen sind, um so ein möglichst intensives Fluoreszenzlicht zu bewirken. Dafür ist, wie schon erwähnt, eine Kugelform der vorzugsweise zudem aus Latex bestehenden Mikropartikel besonders geeignet. Dann könnte für die Detektion dieses angeregten Lichts ggf. ein Lichtschwellenwert definiert werden, der von ggf. in der Probe enthaltenen, durch Anregung aber nur schwach fluoreszierenden sonstigen Bestandteilen nicht überschritten wird und diese somit nicht detektiert und erfasst werden würden.

Bisherige Fluoreszenz-Verfahren basieren auf dem gleichen biochemischen Prinzip, verwenden aber eben eine Trägermatrix mit starrer Plastikoberfläche, z. B. in Mikrotiter-Platten, oder ggf. hochintegrierte "BioChips" mit kompliziertem Aufbau.

Es ist auch denkbar, das erfindungsgemäße Substanz-Feststellungsverfahren auf z. B. hierfür in den menschlichen Körper eingeführte präparierte Mikropartikel anzuwenden, ggf. aber auch erst, nachdem diesem entsprechende Flüssigkeitsproben entnommen oder hybridisierte Mikropartikel aus auf natürlichem Weg ausgeschiedenen Flüssigkeiten wieder zurückgewonnen wurden.

Es soll nicht unerwähnt bleiben, dass das ggf. natürliche Vorkommen von festzustellenden Substanzen in spezifischen Flüssigkeiten, wie z. B. Blut, aber auch in sonstigen, z. B. durch Verschmutzung oder sonstwie verseuchten, erfindungsgemäß zu analysierende Flüssigkeiten - oder auch Gasen - es erfordern kann, die präparierten Mikropartikel nach darin mit entsprechenden Substanzmolekülen oder sonstigen Substanz-Spezifika erfolgter Hybridisierung erst wieder aus der betreffenden Probe zurückzugewinnen, z. B. durch Waschen, Filtrieren oder auch Zentrifugieren, um sie danach erst in ein für das erfindungsgemäße Analysieren durch Größen- und ggf. Fluoreszenslicht-Messung geeignetes reines Substrat entsprechender Viskosität zu geben.

Außer dem Messen der Größe der den Messkanal ausreichend vereinzelt passierenden Mikropartikel können diese auch gleichzeitig noch gezählt werden, um so z. B. eine Information über die Konzentration einer als in der Probe vorhanden detektierten bestimmten Substanz zu erhalten.

Mit dem erfindungsgemäßen Verfahren können durch Wegfall bislang sonst notwendiger zeitaufwändiger, kompliziert durchzuführenden Maßnahmen mittels gezieltem Einsatz präparierter Mikropartikel zum Aufspüren von in einer Probe vermuteten Substanzen erheblich Zeit und Material eingespart werden, wie auch der Geräteaufwand verringert werden kann.

## Patentansprüche

1. Verfahren zum Feststellen von Substanzen in einer flüssigen oder gasförmigen Probe mit den folgenden Schritten, in denen:
- eine der Anzahl der in der Probe vermuteten Substanzen entsprechende Anzahl unterschiedlicher Größenklassen von so genannten Mikropartikeln quasi abstrakt zugeordnet wird,
- Mengen dieser so ausgewählten, größenklassenweise mit jeweils einer der vermuteten Substanzen entsprechenden so genannten Bindungspartnern besetzten Mikropartikel in die Probe gegeben werden, in der in einer sich anschließenden Hybridisierungsphase die einzelnen Bindungspartner der Mikropartikel zuordnungsgemäß jeweils ein Spezifikum der in der Probe tatsächlich vorhandenen Substanz an sich binden, wodurch die Ausgangsgrößen dieser Mikropartikel zunehmen,
- danach die Probe in einer Messküvette durch einen Messkanal mit einer ein vereinzeltes Passieren von Mikropartikeln ermöglichenden Ausgestaltung geleitet wird, wobei ein den Messkanal beleuchtender Laserlichtstrahl in Abhängigkeit von der Größe der durch den Messkanal nacheinander hindurchtretenden hybridisierten bzw. nicht hybridisierten Mikropartikel unterschiedlich stark geschwächt wird, was von einem Lichtschwächungs-Messsensor detektiert und in ein der jeweiligen Schwächung entsprechendes Mikropartikel-Größensignal umgesetzt wird und
- die Messwerte der Mikropartikel-Größensignale in einer Auswertungseinrichtung mit den Größenwerten der Mikropartikel-Ausgangsgrößenklassen verglichen und bei einer letzteren gegenüber ermittelten Größenzunahme anhand der eingangs vorgenommenen Zuordnung von Mikropartikel-Größenklassen zu in der Probe vermuteten Substanzen die in der Probe tatsächlich enthaltenen Substanzen festgestellt sind.

2. Verfahren nach Anspruch 1, **dadurch gekennzeichnet, dass** als Information über das Ergebnis einer Probenanalyse die Namen der festgestellten Substanzen am Analysegerät angezeigt werden.

3. Verfahren nach Anspruch 1 oder 2, **dadurch gekennzeichnet, dass** nach der Hybridisierungsphase in einer Art Waschvorgang nicht an Bindungspartner gebundene Substanz-Spezifika aus der Probe entfernt werden.

4. Verfahren nach Anspruch 1, 2 oder 3, **dadurch gekennzeichnet, dass** für eine zusätzliche Größenzunahme der durch Hybridisierung aus jeweils einem Mikropartikel und an dessen Bündnispartner gebundenen Substanz-Spezifika gebildeten Konstrukte in die bereits hybridisierte Probe Mengen von dementsprechenden Bündnispartnern eingebracht werden für deren Anbinden in einer weiteren Hybridisierungsphase an nunmehr die zuvor an die Bindungspartner dieser Mikropartikel gebundenen Spezifika.

5. Verfahren nach einem der vorhergehenden Ansprüche, **dadurch gekennzeichnet, dass** zum Feststellen von Krankheitserregern diesen entsprechende Antikörper als Bindungspartner benutzt werden.

6. Verfahren nach einem der vorhergehenden Ansprüche, **dadurch gekennzeichnet, dass** zum Feststellen von Antikörpern diesen entsprechende Krankheitserreger als Bindungspartner benutzt werden.

7. Verfahren nach einem der vorhergehenden Ansprüche, **dadurch gekennzeichnet, dass** die Mikropartikel außer für eine Größenzunahme während der Hybridisierungsphase auch noch für ein Aussenden von Licht nach erfolgter Hybridisierung präpariert werden.

8. Verfahren nach Anspruch 7, **dadurch gekennzeichnet, dass** die Mikropartikel für das Aussenden von Fluoreszenslicht präpariert werden.

9. Verfahren nach Anspruch 8, **dadurch gekennzeichnet, dass** hierfür die Mikropartikel mit "Marker" genannten Teilchen beladen werden.

10. Verfahren nach einem der Ansprüche 7 bis 9, **dadurch gekennzeichnet, dass** ein weiterer, den Messkanal beleuchtender Lichtstrahl vorgesehen ist, der beim Auftreffen auf ein hybridisiertes Mikropartikel das Aussenden eines markerdefinierten Fluoreszenzlichts bewirkt, das durch einen diesbezüglichen Fluoreszenslicht-Messsensor detektiert und als Fluoreszenzlicht-Signal ebenfalls an eine Auswertungseinrichtung weitergeleitet wird.

11. Verfahren nach Anspruch 9, **dadurch gekennzeichnet, dass** die beiden jeweils aufgrund ein- und desselben hybridisierten Mikropartikels detektierten Mikropartikelgrößen- und Fluoreszenzlicht-Signale in einer Auswertungseinrichtung einander zugeordnet, die Werte der Mikropartikel-Größensignale mit den Größenwerten der Mikropartikel-Ausgangsgrößenklassen verglichen werden und bei einer letzteren gegenüber festgestellten Größenzunahme anhand der eingangs vorgenommenen Zuordnungen von Mikropartikel-Größenklassen zu in der Probe vermuteten Substanzen die in der Probe tatsächlich enthaltenen Substanzen zumindest für weitere Informationsvorgänge festgestellt werden.

12. Verfahren nach einem der vorhergehenden Ansprüche 6 bis 10, **dadurch gekennzeichnet, dass** das Aussenden von Fluoreszenzlicht von dem für die Größenmessung der Mikropartikel eingesetzten Lichtstrahl bewirkt wird.

13. Verfahren nach Anspruch 12, **dadurch gekennzeichnet, dass** es sich bei dem Lichtstrahl um einen Laserlichtstrahl handelt.

14. Verfahren nach einem der vorhergehenden Ansprüche, **dadurch gekennzeichnet, dass** es sich um zwei verschiedene Laser-lichtstrahlen handelt, die sich zudem in der Wellenlänge unterscheiden können.

15. Verfahren nach Anspruch 14, **dadurch gekennzeichnet, dass** mit Fluoreszenzlicht unterschiedlicher Wellenlängen gearbeitet wird.

16. Verfahren nach Anspruch 1, **dadurch gekennzeichnet, dass** die Mikropartikel aus so genanntem Latex bestehen.

17. Verfahren nach Anspruch 16, **dadurch gekennzeichnet, dass** die Mikropartikel kugelförmig ausgebildet sind und vorzugsweise einen Durchmesser zwischen 2 µm und 150 µm aufweisen.

## Claims

1. A method for the determination of substances in a liquid or gaseous sample, comprising the following steps, in which:
- in a quasi-abstract manner a number of different size classes of micro-particles are assigned to the corresponding number of substances assumed to be in the sample,
- amounts of so selected micro-particles provided with binding partners of one of the assumed substances based on the size classes are added to the sample, wherein in the sample in a subsequent hybridization phase the individual binding partners of the micro-particles bind themselves in accordance with the assignment each to a substance-specific agent of the substance actually present in the sample such that the sizes of those micro-particles increase,
- the sample is conducted subsequently in a measuring cuvette through a measuring channel of a configuration which permits the individual passage of micro-particles, whereby a light beam passing through the measuring channel is differently attenuated depending on the size of the hybridized or non-hybridized micro-particles passing in succession through the measuring channel, which is detected by a light attenuation sensor and converted into a micro-particle size signal corresponding to the respective attenuation, and
- the values of the microparticle size signals are compared in an evaluation unit with the size values of the original micro-particles size classes and, with the determination of an increase in the size of the micro-particles over the size class values in accordance with the original assignment of micro-particle size classes determining the presence of the assumed substances in the sample.

2. A method according to claim 1, **characterized in that** as information concerning the result of a sample analysis, the names of the substances determined to be present in the sample are indicated on the analysis apparatus.

3. A method according to claim 1 or 2, **characterized in that** after the hybridization phase substance-specific agents which are not bound to a binding partner are removed from the sample in a washing procedure.

4. A method according to claim 1, 2 or 3 **characterized in that** for an additional size increase of constructs formed by the hybridization of a micro-particle and substance-specific agents bound to its binding partner, amounts of corresponding binding partners are added to the already hybridized sample for the binding thereof in an additional hybridization phase to the substance-specific agents bound earlier to the binding partners of these micro-particles.

5. A method according to one of the preceding claims, **characterized in that** for the determination of pathogens, antibodies corresponding to those pathogens are used as binding partners.

6. A method according to one of the preceding claims, **characterized in that** for the determination of antibodies, the corresponding pathogens are used as binding partners.

7. A method according to one of the preceding claims, **characterized in that** said micro-particles, in addition to their preparation during the hybridization phase for a size increase are also prepared for the emission of light after the hybridization is completed.

8. A method according to claim 7, **characterized in that** the micro-particles are prepared for the emission of fluorescence light.

9. A method according to claim 8, **characterized in that** for the emission fluorescence light, the micro-particles are charged with "marker" particles.

10. A method according to one of claims 7 to 9, **characterized in that** a further light beam illuminating the measuring channel is provided which, upon striking a hybridized particle, initiates the emission of a marker-defined fluorescence light, which is detected by a respective fluorescence light measurement sensor and is transmitted as a fluorescence light signal also to the evaluation device.

11. A method according to claim 9, **characterized in that** the values of the micro-particle size and the fluorescence light signals which are detected on the basis of the same hybridized particles are compared with the size classes of the initial micro-particles size classes and, if it is found that the size of the micro-particles has been increased over that of the initial micro-particle size classes based on the initial assignment of micro-particle size classes to substances assumed to be present in the sample, the substances actually present in the sample are determined for further information procedures.

12. A method according to one of the preceding claims 6 to 10, **characterized in that** the emission of fluorescence light is caused by the light beam used for the size measurements of the micro-particles.

13. A method according to claim 12, **characterized in that** the light beam is a laser light beam.

14. A method according to one of the preceding claims, **characterized in that** two different laser light beams are provided which additionally may have different wavelengths.

15. A method according to claim 14, **characterized in that** fluorescence light of different wavelength is used.

16. A method according to claim 1, **characterized in that** the micro-particles consist of so-called latex.

17. A method according to claim 16, **characterized in that** micro-particles are spherical and have preferably a diameter between 2 µm and 150 µm.

## Revendications

1. Procédé de détermination de substances dans un échantillon liquide ou gazeux, comprenant les étapes consistant à :
- affecter de manière quasi abstraite un nombre de classes de tailles différentes de ce que l'on appelle des microparticules, correspondant au nombre des substances supposées dans l'échantillon,
- ajouter des quantités desdites microparticules ainsi sélectionnées par classes de tailles, occupées par ce que l'on appelle des partenaires de liaison qui correspondent chacun à l'une des substances supposées, à l'échantillon où, dans une phase d'hybridation consécutive, les différents partenaires de liaison des microparticules se lient chacun en fonction de leur destination à un élément spécifique de la substance effectivement présente dans l'échantillon, ce qui fait augmenter les tailles de sortie de ces microparticules,
- faire passer ensuite l'échantillon dans une cuvette de mesure par un conduit de mesure ayant une conformation qui permet le passage une à une des microparticules, un faisceau de lumière laser éclairant le conduit de mesure étant plus ou moins fortement atténué en fonction de la taille des microparticules hybridées ou non hybridées qui pénètrent les unes à la suite des autres dans le conduit de mesure, ce qui est détecté par un capteur de mesure d'atténuation de la lumière et converti en un signal de taille de microparticules correspondant à chaque atténuation, et
- comparer dans un dispositif d'évaluation les valeurs de mesure des signaux de taille de microparticules avec les valeurs de taille des classes de taille de départ des microparticules et, en présence d'une augmentation de taille détectée par rapport à ces dernières, déterminer, au moyen de l'affectation des classes de tailles de microparticules effectuée initialement pour les substances supposées dans l'échantillon, les substances qui sont effectivement contenues dans l'échantillon.

2. Procédé selon la revendication 1, **caractérisé en ce que**, comme information concernant le résultat d'une analyse d'échantillon, les noms des substances déterminées sont affichés sur l'appareil d'analyse.

3. Procédé selon la revendication 1 ou 2, **caractérisé en ce que**, après la phase d'hybridation, les éléments spécifiques de la substance qui ne sont pas liés à des partenaires de liaison sont enlevés de l'échantillon par une sorte d'opération de lavage.

4. Procédé selon la revendication 1, 2 ou 3, **caractérisé en ce que**, pour une augmentation supplémentaire de la taille des produits d'assemblage formés chacun d'une microparticule et des éléments spécifiques d'une substance liés par hybridation à ses partenaires de liaison, des quantités de partenaires de liaison correspondants sont introduites dans l'échantillon déjà hybridé en vue de leur rattachement dans une autre phase d'hybridation à des éléments spécifiques préalablement liés aux partenaires de liaison de ces microparticules.

5. Procédé selon l'une des revendications précédentes, **caractérisé en ce que**, pour déterminer des agents pathogènes, on utilise comme partenaires de liaison des anticorps qui leur correspondent.

6. Procédé selon l'une des revendications précédentes, **caractérisé en ce que**, pour déterminer des anticorps, on utilise comme partenaires de liaison des agents pathogènes qui leur correspondent.

7. Procédé selon l'une des revendications précédentes, **caractérisé en ce que** les microparticules sont préparées également, en plus d'une augmentation de taille pendant la phase d'hybridation, pour émettre de la lumière une fois l'hybridation effectuée.

8. Procédé selon la revendication 7, **caractérisé en ce que** les microparticules sont préparées pour émettre de la lumière fluorescente.

9. Procédé selon la revendication 8, **caractérisé en ce que**, pour cela, les microparticules sont chargées avec des particules dites de "marquage".

10. Procédé selon l'une des revendications 7 à 9, **caractérisé en ce qu'**il est prévu un autre faisceau lumineux éclairant le conduit de mesure qui, lorsqu'il vient frapper une microparticule hybridée, provoque l'émission d'une lumière fluorescente, définie par un marqueur, qui est détectée par un capteur de mesure de lumière fluorescente et transmise également sous la forme d'un signal de lumière fluorescente à un dispositif d'évaluation.

11. Procédé selon la revendication 9, **caractérisé en ce que** les deux signaux de taille de microparticules et de lumière fluorescente détectés chacun à partir d'une même microparticule hybridée sont affectés l'un à l'autre dans un dispositif d'évaluation, les valeurs des signaux de taille de microparticules sont comparées aux valeurs de taille des classes de taille initiale de microparticules et, en présence d'une augmentation de taille détectée par rapport à ces dernières, les substances effectivement contenues dans l'échantillon sont déterminées à partir des affectations de classes de taille de microparticules effectuées initialement pour les substances supposées dans l'échantillon, au moins pour d'autres processus d'information.

12. Procédé selon l'une des revendications 6 à 10 précédentes, **caractérisé en ce que** l'émission de lumière fluorescente est provoquée par le faisceau lumineux utilisé pour mesurer la taille des microparticules.

13. Procédé selon la revendication 12, **caractérisé en ce que** le faisceau lumineux est un faisceau lumineux laser.

14. Procédé selon l'une des revendications précédentes, **caractérisé en ce qu'**il s'agit de deux faisceaux lumineux laser différents, qui peuvent différer en plus par leur longueur d'onde.

15. Procédé selon la revendication 14, **caractérisé en ce que** l'on travaille avec de la lumière fluorescente de différentes longueurs d'onde.

16. Procédé selon la revendication 1, **caractérisé en ce que** les microparticules se composent de ce que l'on appelle du latex.

17. Procédé selon la revendication 16, **caractérisé en ce que** les microparticules ont une forme sphérique et présentent de préférence un diamètre dans la plage de 2 µm à 150 µm.
